(19) Europäisches Patentamt
European Patent Office
Office européen des brevets

(11) **EP 1 380 251 B1**

(12) **EUROPÄISCHE PATENTSCHRIFT**

(45) Veröffentlichungstag und Bekanntmachung des Hinweises auf die Patenterteilung:
**03.09.2008 Patentblatt 2008/36**

(51) Int Cl.:
*A61B 3/024* *(2006.01)*     *A61B 3/00* *(2006.01)*

(21) Anmeldenummer: **03405577.2**

(22) Anmeldetag: **05.08.2003**

(54) **Projektionsperimeter**

Projection perimeter

Perimètre à projection

(84) Benannte Vertragsstaaten:
**CH DE FR GB IT LI**

(43) Veröffentlichungstag der Anmeldung:
**14.01.2004 Patentblatt 2004/03**

(73) Patentinhaber: **HAAG-STREIT AG**
**CH-3098 Köniz (CH)**

(72) Erfinder: **Scharf, Klaus-Dieter**
**78126 Köngsfeld-Erdmannsweiler (DE)**

(74) Vertreter: **Roshardt, Werner Alfred et al**
**Keller & Partner**
**Patentanwälte AG**
**Schmiedenplatz 5**
**Postfach**
**3000 Bern 7 (CH)**

(56) Entgegenhaltungen:
**EP-A- 1 114 608**     **US-A- 5 220 361**
**US-A- 5 235 360**     **US-A1- 2003 007 128**
**US-B1- 6 183 086**

- **PATENT ABSTRACTS OF JAPAN vol. 016, no. 369 (C-0972), 10. August 1992 (1992-08-10) -& JP 04 117941 A (TOPCON CORP), 17. April 1992 (1992-04-17)**
- **PATENT ABSTRACTS OF JAPAN vol. 1996, no. 07, 31. Juli 1996 (1996-07-31) & JP 08 066359 A (TOPCON CORP), 12. März 1996 (1996-03-12)**
- **PATENT ABSTRACTS OF JAPAN vol. 017, no. 638 (C-1133), 26. November 1993 (1993-11-26) -& JP 05 199996 A (CANON INC), 10. August 1993 (1993-08-10)**
- **PATENT ABSTRACTS OF JAPAN vol. 018, no. 285 (C-1206), 31. Mai 1994 (1994-05-31) -& JP 06 054804 A (CANON INC), 1. März 1994 (1994-03-01)**

EP 1 380 251 B1

**Beschreibung**

**Technisches Gebiet**

Die Erfindung betrifft ein Projektionsperimeter zur Untersuchung eines Gesichtsfeldes eines Patienten. Die Erfindung ist in der statischen und kinetischen Perimetrie anwendbar und ermöglicht eine flexible Durchführung einer Gesichtsfelduntersuchung in einer Hohlkugel, der so genannten Kupola.

**Stand der Technik**

Bei den bekannten Perimetem wird ein Lichtfleck mit definierter Grösse und Beleuchtungsstärke auf eine mattweisse Kugelinnenoberfläche einer Kupola abgebildet. Der projizierte Stimulus kann dabei weiss oder auch farbig sein. Das Perimeter muss die Möglichkeit bieten, verschiedene Stimulusgrössen in der Weise zu projizieren, dass der jeweilige Raumwinkel, unter dem der Patient den Stimulus wahrnimmt, in allen vorgeschriebenen Positionen auf der Kugelinnenfläche innerhalb einer bestimmten Toleranz (+20% sowie - 15% entsprechend ISO 12866) bleibt.

In der US-A 5,235,360 ist ein Perimeter mit einer hemisphärischen Kupola beschrieben, welches mit einer konventionellen Lichtquelle arbeitet. Als konventionelle Lichtquelle wird ein so genannter "Light-spot" verwendet, dessen Helligkeit über ein Graufilter einstellbar ist. Ausserhalb des Kupolamittelpunkts, jedoch innerhalb der Kupola, aber innerhalb des standardisierten Gesichtsfeldes befindet sich eine Schwenkspiegelanordnung, mit der der Lichtstrahl der konventionellen Lichtquelle ("Light-spot") auf vorgegebene Orte auf einer Innenfläche der Kupola als Stimulus zum Ausmessen eines Gesichtsfeldes eines Auges des Patienten projizierbar ist.

Aus der EP-A 242 723 ist ein Projektionsperimeter bekannt dessen Kupola einen oberen flachen Bereich mit einer Durchbruchsöffnung aufweist. Das bekannte Perimeter hat eine ausserhalb der Kupola angeordnete Halogenleuchte, die mit einer Farbtemperatur von 2'900°C arbeitet. Die Strahlung der Halogenleuchte wird mit einer ersten Linse auf eine Anordnung mit einem Filter und einem Blendenrad sowie einem Verschluss geführt. Die austretende Strahlung wird mit einem ersten stationären Spiegel umgelenkt und mit einem Objektiv nach Durchlaufen eines optischen Systems auf die Innenseite der Kupola abgebildet. Nach dem Objektiv ist ein zweiter feststehender Spiegel vorhanden, der den Strahl in eine Weglängenvariationsanordnung mit zwei zueinander starr angeordneten, aber zusammen verschiebbaren Spiegeln richtet. Die Weglängenvariationsanordnung ist derart ausgebildet, dass der ein- und der austretende Strahl parallel zueinander verlaufen. Durch Verschieben dieser beiden Spiegel miteinander gegenüber dem zweiten Spiegel und dem nachfolgend erwähnten dritten Spiegel, ist die optische Weglänge zwischen dem zweiten Spiegel und dem entsprechenden Ort des Stimulus veränderbar. Der aus der Weglängen verändernden Spiegelanordnung austretende Strahl wird auf einen dritten feststehenden Spiegel geführt, durchläuft ein um seine Achse verschwenkbares Periskop und trifft in der Nähe der Durchbruchsöffnung, deutlich distanziert von der Stirn des Patienten, auf einen Schwenkspiegel. Der Schwenkspiegel ist um eine Achse senkrecht zur Periskopachse verschwenkbar. Durch diese beiden Schwenkrichtungen kann der Lichtstrahl auf unterschiedliche Orte der Kupolainnenseite gerichtet werden. Da die optischen Wege zwischen Schwenkspiegel und Auftreffort auf der Innenseite unterschiedlich lang sind, muss eine Längenkorrektur des Lichtstrahlweges durch die Weglängenvariationsanordnung vorgesehen werden.

**Aufgabe der Erfindung**

Aufgabe der Erfindung ist es, ein Projektionsperimeter in einer einfachen, preisgünstigen Bauart zu schaffen, wobei in einer weiteren Aufgabe gefordert wird, dass der Stimulus auf der Innenseite der Kupola nur geringfügig von einer Kreisform abweicht. Eine preisgünstige Bauart schliesst im Augenblick die Verwendung eines Lasers als Lichtquelle sowie die Verwendung eines einstellbaren Zoomobjektivs aus.

**Lösung der Aufgabe**

Die Aufgabe wird dadurch gelöst, dass ein Projektionsperimeter mit einem Kopfhalter für einen Patientenkopf, mit einer Kupola, mit wenigstens einer Lichtquelle und mit einer ausserhalb des Kupolamittelpunkts angeordneten Schwenkspiegelanordnung verwendet wird. Von der Lichtquelle ist ein Lichtstrahl aussendbar, der mit der Schwenkspiegelanordnung auf vorgegebene Orte auf einer Innenfläche der Kupola als Stimulus zum Ausmessen eines Gesichtsfeldes eines Auges des Patienten führbar ist. Die Lichtquelle hat nun erfindungsgemäss wenigstens eine Licht-Emittierende-Diode (LED).

Die EP-A 0 242 723 benützte als Lichtquelle eine Halogenstrahlungsquelle mit einer Farbtemperatur von 2'900°C. Eine derartige Strahlungsquelle durfte schon aufgrund ihrer auch noch bei Kühlung hohen Temperatur nicht allzu nah am Patientenkopf angeordnet werden. Aufgrund der zu verwendenden Filter für unterschiedliche Farbeindrücke des Stimulus und des "Temperaturabstandes" ergab sich eine voluminöse Konstruktion.

Die Erfindung hingegen schlägt vor, eine Leuchtdiode [Licht-Emittierende-Diode (LED)] zu verwenden. Eine Vielzahl von LEDs wurden zwar in Perimetern schon verwendet, jedoch nur in einer netzartigen Anordnung als Punktlichtquellen festmontiert in der Innenseite der Kupola (Perimeter mit festem Prüfraster). Bei Projektionsperimetern eine einen Lichtstrahl aussendende LED zu verwenden, wobei der Lichtstrahl mit einer Schwenkspiegelanordnung an Orte auf der Kupolainnenseite projiziert wurde, war der Fachwelt fremd. Wie in der Detailbeschreibung ausgeführt, ist es entgegen der übliche Meinung der Fachwelt jedoch möglich, auch mit einer Leuchtdiode die für die Bestimmung des Gesichtsfeldes geforderten Beleuchtungsstärken im Stimulus zu erhalten.

Eine Leuchtdiode ist nicht nur kleiner und kälter als eine Halogenstrahlungsquelle; sie ist auch bedeutend erschütterungsresistenter; sie kann somit problemlos auch mit hohen Beschleunigungen und damit kurzen Einstellzeiten verfahren werden.

Anstelle einer einzigen Leuchtdiode können selbstverständlich auch mehrere, wie unten angeführt, verwendet werden, die in unterschiedlichen Farben leuchten. Diese Leuchtdioden wird man dann auf einer Wechseleinrichtung, wie z.B. einem Revolver, anordnen. Die einzelnen Dioden können dann jeweils einzeln zur Erzeugung eines Lichtstrahls mit nur einer einzigen Farbe verwendet werden. Man kann aber auch mehrere Dioden zusammen strahlen lassen und ihre Ausgangsstrahlung zu einem den Stimulus erzeugenden Strahl zusammenfassen; hiermit lassen sich dann Mischfarben und sogar weisses Licht erzeugen.

Wie mit Leuchtdioden eine ausreichende Leuchtdichte von wenigstens 4'000 asb im Stimulus erreichbar sind, ist in der Detailbeschreibung ausgeführt. Wird mit einer Umfeldleuchtdichte von 31,4 asb in der Kupola gearbeitet, so sollte eine Leuchtdichte von wenigstens 4'000 asb im Stimulus erreicht werden. Zum Erreichen dieser Leuchtdichte bei einem Projektionsperimeter wurde bisher mit z.B. Halogenleuchten gearbeitet. Eine Leuchtdiode wurde nicht verwendet. Wird in nahezu vollkommener Dunkelheit in der Kupola bei einer Umfeldleuchtdichte von 4 asb gearbeitet, reichen bereits 1'000 asb Leuchtdichte des Stimulus aus.

Die weitere Aufgabe, bei einer einfachen Bauart einen Stimulus zu erzeugen, der auf der Innenseite der Kupola nur eine von der Kreisform geringfügige Abweichung hat, wird dadurch gelöst, dass ein Projektionsperimeter geschaffen wird, dass eine Kupola, eine ausserhalb des Kupolamittelpunkts angeordnete Schwenkspiegelanordnung, einen Kopfhalter für einen Patientenkopf und wenigstens eine Lichtquelle hat. Von der Lichtquelle ist ein Lichtstrahl aussendbar, der mit der Schwenkspiegelanordnung auf vorgegebene Orte auf einer Innenfläche der Kupola als Stimulus zum Ausmessen eines Gesichtsfeldes eines Auges des Patienten führbar ist. In einer Ausführungsvariante ist die Schwenkspiegelanordnung ausserhalb eines räumlichen Standardgesichtsfeldes eines Patientenauges dicht an dessen Aussengrenze derart angeordnet, dass die optische Achse des auf die Schwenkspiegelanordnung treffenden Lichtstrahls gegenüber der Kupolaachse einen Winkel grösser als 40°, bevorzugt grösser als 50°, hat. Hierdurch ergeben sich auf der Kupolainnenfläche zwischen der optischen Achse des auftreffenden Lichtstrahls und der Geraden zum Patientenauge im Gegensatz zum Stand der Technik kleinere Winkel. Je kleiner dieser Winkel ist, desto kleiner ist die Abweichung von der idealen Kreisform (kleinere Exzentrizität). Diese Ausführungsvariante wird man vorzugsweise mit einer Leuchtdiode oder mehreren Leuchtdioden als Lichtquelle betreiben; sie kann aber mit denselben Vorteilen auch mit einer thermischen Lichtquelle (Halogenstrahlungsquelle, ...) betrieben werden. Zum Vergleich sind diese Winkel $\alpha_1$ und $\alpha_2$ in Figur 12 eingetragen. Figur 12 ist die **Figur 5** der bereits oben zitierten EP-A 0 242 723.

Mit der hier beschriebenen Strahlführung kann eine Abweichung von der kreisförmigen Sollfläche des Stimulus von lediglich 5% erreicht werden. Da der Abstand (vom Patientenauge zum Stimulus) bei der Berechnung des Raumwinkels zum Quadrat eingeht, bleiben bei dem in der Detailbeschreibung erläuterten optischen Aufbau Abweichungen des sich ergebenden Raumwinkels, unter dem der Patient den Stimulus beobachtet, innerhalb einer Toleranz von +/- 14%, d. h. die Abweichungen liegen bei lediglich +/-12,5% vor. Die hier beschriebene Ausführungsvariante der Erfindung kommt somit einem idealen Messaufbau für ein Ausmessen des Gesichtsfeldes recht nahe; die von Goldmann angegebene Forderung eines runden Prüfreizes ist somit innerhalb einer Messtoleranz eingehalten.

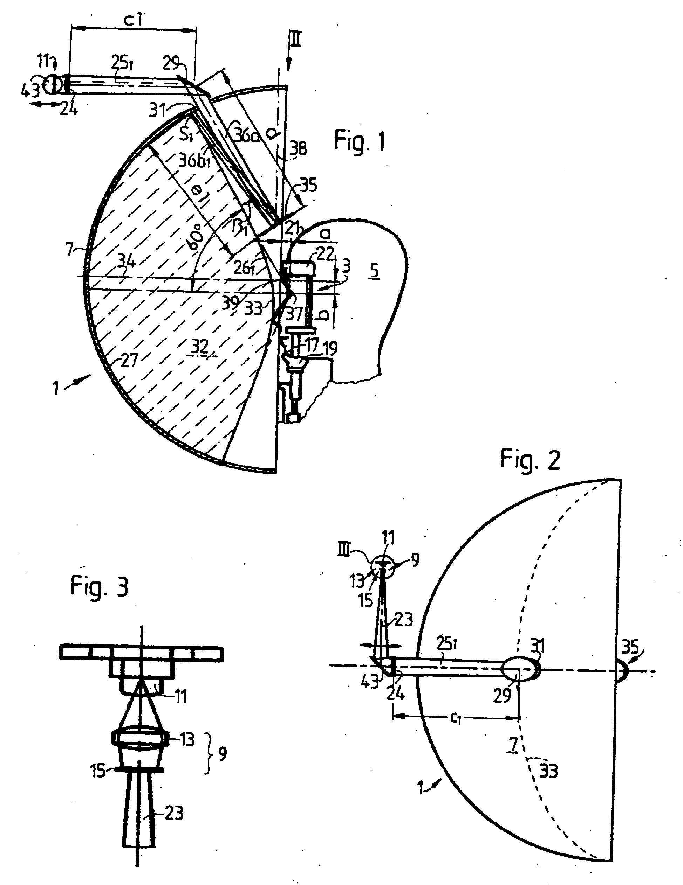

Die optische Achse des auf die Schwenkspiegelanordnung treffenden Lichtstrahls gegenüber der Kupolaachse (in den Figuren 1, 2 und 4 bis 11 eine Horizontale durch den Kupolamittelpunkt gehende Gerade) soll bei der Erfindung einen Winkel grösser als 40°, bevorzugt grösser als 50°, aufweisen. Die EP-A 0 242 723 als Stand der Technik hat im Gegensatz hierzu einen deutlich kleineren Winkel von lediglich 27°.

Die Lichtquelle wird man vorzugsweise ausserhalb der Kupola anordnen und deren den Stimulus erzeugenden Lichtstrahl durch einen Durchbruch in der Kupolawand hindurchführen. Der Durchbruch ist ausserhalb des Gesichtsfeldes, jedoch dicht an der Gesichtsfeldgrenze angeordnet. Die Grösse des Durchbruchs ist bei der Wahl von Leuchtdioden als Lichtquelle wichtig.

Die Grenze des Gesichtsfelds eines "Standard"-Patienten liegt bei ungefähr 60° gegen oben und 70° nach unten; seitlich liegt die Grenze bei etwa 85°. In der bereits oben zitierten EP-A 0 242 723 ist ein als Schwenkspiegel wirkender Spiegel (siehe dortige Figuren 2 und 3) weit oberhalb der mit dem Kopfhalter fixierten Patientenstirn innerhalb der Kupola angeordnet.

Im Gegensatz zur EP-A 0 242 723 wird hier der Abstand der Schwenkspiegelanordnung vom Mittelpunkt der Kupola klein gehalten. Durch eine Schwenkspiegelanordnung in der Nähe des Kupolamittelpunktes ergeben sich einerseits nur geringe Weglängenunterschiede von der Schwenkspiegelanordnung zum Ort des Stimulus, welche korrigiert werden müssen, und andererseits ist eine Verzeichnung von einem kreisförmigen Querschnitt des Stimulus gering. Der Patient sieht nahezu, d.h. bis auf eine kleine zu tolerierende Abweichung, immer einen Kreis. Ein genereller Schwenkort der Schwenkspiegelanordnung ist oberhalb des Kupolamittelpunkts in einem Abstand von lediglich 15% bis 50% des Kupoladius, bevorzugt bei 20% bis 35%, d.h. in unmittelbarer Nähe zum Ort der mit dem Kopfhalter fixierbaren Patientenstirn angeordnet.

In der Regel wird nach der Messmethode von Goldman gearbeitet, wodurch sich ein Radius der Kupola von 300 mm ergibt.

Unter einem generellen Schwenkort wird der Ort einer theoretischen Schwenkachse verstanden. Die Schwenkspiegelanordnung kann beispielsweise als ein um seine Achse verschwenkbares "Periskop" mit einem zur Periskopachse eine senkrechte Schwenkachse aufweisenden Spiegel ausgebildet sein. Der generelle Schwenkort ist dann der Schnittpunkt der beiden zueinander senkrecht stehenden Schwenkachsen.

Anstelle eines "Periskops" mit Schwenkspiegel können auch zwei Galvanometerspiegelanordnungen dienen, deren Spiegelschwenkachsen senkrecht zueinander liegen, wobei dann der eine Spiegel eine Ablenkung in einer Ebene und der andere eine Ablenkung in der anderen, hierzu senkrechten Ebene vornimmt, sodass eine Fläche bedeckt werden kann. Ein genereller Schwenkort liegt dann zwischen den beiden Schwenkachsen.

Die EP-A 0 242 723 konstruierte ihre Kopfhalterung derart, dass das Patientenauge im Mittelpunkt der Kupola zu liegen kam. Zum Ausmessen eines räumlichen Gesichtsfeldes auf einer Kugelinnenseite der Kupola schien dies der genau richtige Weg zu sein. Die Abstände vom Patientenauge zu den in verschiedenen Richtungen und Orten projizierten Stimuli waren dann gleich. Die Erfindung hingegen schlägt in einer Ausführungsvariante einen hiervon abweichenden Weg vor.

Der Kopfhalter der Erfindung ist nun derart ausgebildet, dass der Patientenkopf derart lagerbar ist, dass das zu untersuchende Patientenauge eine aussermittig, vorzugsweise nach unten, versetzte Lage zum Kupolamittelpunkt hat, damit in Zusammenwirkung mit der aussermittigen Schwenkspiegelanordnung jeweils ein Winkel zwischen dem auf der Innenseite der Kupola auftreffenden Lichtstrahl und einer Beobachtungsachse zum Patientenauge klein gehalten werden kann, um hierdurch eine lageabhängige Verzeichnung des Stimulusquerschnitts ebenfalls klein zu halten. Dieser Effekt lässt sich durch einen zusätzlichen Versatz des Patientenauges aus dem Kupolamittelpunkt nach aussen noch verbessern.

Das Patientenauge wird man aussermittig zum Kupolamittelpunkt, nach unten, vorzugsweise um 5% bis 10% bezogen auf den Kupoladius, versetzt anordnen. Der Versatz nach aussen liegt zwischen 3% bis 8% bezogen auf den Kupoladius.

Die zum Kugelzentrum exzentrisch verlaufende Strahlführung zum Stimulus bewirkt, wenn auch nur im Falle der Erfindung geringe, unterschiedliche Strahlwege in Bezug auf unterschiedliche Orte auf der Kupolainnenfläche. Bei unterschiedlichen Abständen zwischen Objektiv und Auftreffpunkt des Lichtstrahls auf der Kugelinnenfläche würden sich bei ein- und derselben Blendengrösse unterschiedlich grosse Stimulusgrössen in Abhängigkeit von der Lage des Stimulus ergeben.

In einer Ausführungsvariante der Erfindung wird nun vorgeschlagen, die Lichtquelle in einer starren Anordnung zu ihrem Strahlformungssystem [Objektiv (Kondensor) + (gegebenenfalls) Blende] in Richtung der optischen Achse des auf die Schwenkspiegelanordnung treffenden Lichtstrahls entsprechend der (geringen) Abstandsänderung zu verfahren.

Hierdurch ergibt sich eine äusserst einfache Konstruktion mit Strahlungsquelle, Kondensor und Blende sowie hieran anschliessend ein Objektiv, welches die Blende auf der Kupolainnenseite abbildet. Das Objektiv zusammen mit Strahlungsquelle, Kondensor und Blende werden nun entsprechend der Lage des Orts des Stimulus verschoben. Wählt man wie oben angedeutet als Strahlungsquelle eine Leuchtdiode [Licht-Emittierende-Diode (LED)] kann problemlos leicht und mit hoher Beschleunigung verschoben (korrigiert) werden, da eine LED gegen Stösse und starke Beschleunigungen sehr unempfindlich ist, d.h. bedeutend unempfindlicher als eine Halogenstrahlungsquelle ist, wie sie beispielsweise die EP-A 0 242 723 verwendet.

Wird zwischen der Blendenanordnung und dem Objektiv ein Umlenkelement (vorzugsweise ein Spiegel oder ein Prisma) angeordnet, der die von der Strahlungsquelle kommende Strahlung um 90° umlenkt, kann eine Reduktion der Baugrösse erreicht werden. Zur optischen Weglängenkompensation bei unterschiedlicher Stimuluslage wird jetzt die Anordnung mit Strahlungsquelle, Kondensor und Blende sowie Objektiv und Umlenkelement als Ganzes bezüglich der Schwenkspiegelanordnung verschoben.

Eine weitere Baugrössenreduktion ist erreichbar, wenn eine Strahl umkehrung nicht wie eben geschildert um 90°, sondern um 180° vorgenommen wird. Strahlungsquelle, Kondensor und Blende sowie Objektiv und Umlenkelement werden auch hier als Ganzes verschoben, können aber ausserhalb der Kupola über dieser angeordnet sein.

Aus der nachfolgenden Detailbeschreibung und der Gesamtheit der Patentansprüche ergeben sich weitere vorteilhafte Ausführungsformen und Merkmalskombinationen der Erfindung.

**Kurze Beschreibung der Zeichnungen**

Die zur Erläuterung der Ausführungsbeispiele verwendeten Zeichnungen zeigen

Fig. 1        einen Längsschnitt durch das erfindungsgemässe Projektionsperimeter, wobei ein Stimulus im Gesichtsfeld unmittelbar an seiner oberen Gesichtsfeldgrenze zu liegen kommt,

Fig. 2        eine Draufsicht auf das in **Figur 1** dargestellte Projektionsperimeter in Blickrichtung II in Figur 1 ohne den in **Figur 1** dargestellten Kopfhalter mit Patitenkopf,

Fig. 3        eine vergrösserte Darstellung des Bereiches III in Figur 1 mit einer Lichtquelle, einem Kondensor und einer Blende,

Fig. 4        eine zu **Figur 1** analoge Darstellung, wobei jedoch ein Stimulus $S_2$ in der Nähe der Kupolaachse zu liegen kommt,

Fig. 5        eine zu **Figur 2** analoge Darstellung einer Draufsicht mit der in **Figur 4** gezeigten Stimuluslage $S_2$ in der in **Figur 4** gezeigten Blickrichtung V,

Fig. 6        einen Längsschnitt durch das in **Figur 1** dargestellte Projektionsperimeter, wobei die Projektion des Stimulus $S_3$ im unteren Extrempunkt liegt,

Fig. 7        eine Draufsicht auf das in **Figur 6** dargestellte Projektionsperimeter in der in **Figur 6** gezeigten Blickrichtung VII,

Fig. 8        eine zu **Figur 1** analoge Ausführung eines Projektionsperimeters, wobei jedoch deren in unmittelbarer Umgebung zum Objektiv angeordnete Umlenkspiegel durch ein den Lichtstrahl um 180° umkehrendes Umkehrprisma zur Platzersparnis ersetzt ist,

Fig. 9        eine Draufsicht auf das in **Figur 9** dargestellte Projektionsperimeter in der in **Figur 8** gezeigten Blickrichtung IX,

Fig. 10      einen Längsschnitt durch ein Projektionsperimeter gemäss **Figur 8,** wobei die Projektion des Stimulus $S_3$ im unteren Extrempunkt liegt,

Fig. 11      eine Draufsicht auf das in **Figur 10** dargestellte Projektionsperimeter in der dortigen Figur gezeigten Blickrichtung XI und

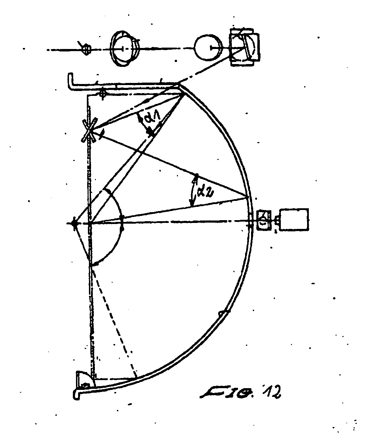

Fig. 12      Figur 5 der EP-A 0 242 723, in die die relativ grossen Winkel $\alpha_1$ und $\alpha_2$ zwischen der optischen Achse des auf die Innenfläche der Kupola treffenden Lichtstrahls und einer Gerade von diesem Auftreffort zum Patientenauge eingezeichnet sind. Grosse Winkel bewirken eine grosse exzentrische Abweisung des Stimulusquerschitts (Die grossen Winkel bewirken eine grosse Abweichung des Stimulusquerschnitts von einer Kreisform).

**Wege zur Ausführung der Erfindung**

Das in den **Figuren 1, 4** und **6** in einem schematischen Längsschnitt und in den **Figuren 2, 5** und **7** in einer Draufsicht dargestellte erfindungsgemässe Projektionsperimeter **1** hat einen Kopfhalter **3** für einen Patientenkopf **5**, eine Kupola **7** und eine ausserhalb der Kupola **7** liegende, ein Strahlformungssystem **9** aufweisende Lichtquelle **11**. Der Kopfhalter **3** mit dem Patientenkopf **5** ist lediglich in **Figur 1** dargestellt. Die Lichtquelle **11** mit dem Strahlformungssystem **9** ist vergrössert in **Figur 3** dargestellt. Das Strahlformungssystem **9** besteht aus einem Kondensor **13** und einer Blendenanordnung **15**. Der Patientenkopf **5** liegt mit seinem Kinn **17** auf einem Kinnpolster **19** auf und mit seiner Stirn **21** an einem Stirnband **22** an. Der von der Lichtquelle **11** ausgesandte Lichtstrahl **23** beleuchtet die Blendenöffnung der Blendenanordnung **15**. Die strahlende Fläche der Lichtquelle **11** wird über den Kondensor **13** und die Blendenöffnung in die Eintrittspupille des Objektivs **24** abgebildet. Die Blendenöffnung wird mit dem Objektiv **24** auf der Innenseite **27** der Kupola **7** in **Figur 1** an einem Ort $S_1$ (in **Figur 1** dargestellt) abgebildet. Der Teillichtstrahl vor dem Objektiv **24** ist mit **23** und derjenige nach dem Objektiv **24** bis zu einem unten erwähnten feststehenden Umlenkspiegel **29** mit **25** gekennzeichnet. Der Teillichtstrahl nach dem Umlenkspiegel **29** bis zu einer unten beschriebenen Schwenkspiegelanordnung **35** ist mit **36a** und von der Schwenkspiegelanordnung **35** zum Ort $S_1$ mit **36b** gekennzeichnet. Die Strahlabbildung auf der Innenseite **27** am Ort **S,** welche je nach Ablenkung durchnummeriert sind, wird Stimulus genannt. Analog sind die Teilstrahlen **36b** in unterschiedlichen Lagen durch Indizes **n** (1, 2, ...) durchnummeriert. Der Lichtstrahl **25** wird nach dem Objektiv **24** mit einem feststehenden Umlenkspiegel **29** durch einen im oberen Bereich der Kupola 7 angeordneten Durchbruch **31** in diese hineingeführt. Der Durchbruch **31** ist ausserhalb des auf die Innenseite **27** der Kupola **7** zu projizierenden Gesichtsfeldes **32** so dicht wie möglich an dessen Aussengrenze **33** angeordnet. Die vertikale Begrenzung des Gesichtsfeldes **32** ist in **Figur 1** und die horizontale Begrenzung in **Figur 2** dargestellt.

Das Gesichtsfeld eines normalsichtigen menschlichen Auges ("Standardauge") beträgt ungefähr 60° nach oben, 70° nach unten und seitwärts 85°, wie in **Figur 2** durch die gestrichelte Linie **33** angedeutet ist.

Eine Schwenkspiegelanordnung **35** ist in unmittelbarer Nähe zum Ort der mit dem Kopfhalter **3** fixierbaren Patientenstirn **21** angeordnet. Die Schwenkspiegelanordnung **35** ist ausserhalb eines räumlichen Standardgesichtsfeldes **32** dicht an dessen Aussengrenze **33** derart angeordnet, dass eine optische Achse **36a** des auf die Schwenkspiegelanordnung **35** treffenden Lichtstrahls gegenüber der Kupolaachse **34** einen Winkel grösser als 40°, bevorzugt grösser als 50°, hat. In der hier gezeigten Ausführungsvariante liegt dieser Winkel bei 57°. Die Schwenkspiegelanordnung **35** ist derart gewählt, dass ihr genereller Schwenkort oberhalb des Kupolamittelpunkts **39** in einem Abstand vom Kupolamittelpunkt **39** von lediglich 15% bis 50% des Kupolaradius, bevorzugt bei 20% bis 35%, d.h. in unmittelbarer Nähe zum Ort, der mit dem Kopfhalter **3** fixierbaren Patientenstirn **21** angeordnet ist.

Die Schwenkspiegel der Schwenkspiegelanordnung **35** sind in **Figur 1** um eine senkrecht zur auftreffenden optischen Strahlachse **36a** und senkrecht zur Zeichenebene angeordnete Schwenkachse sowie um eine weitere in der Zeichnungsebene liegende Schwenkachse **38** verschwenkbar ausgebildet. Mit der Schwenkspiegelanordnung **35** ist der jeweilige geometrische Ort des Stimulus $S_n$ auf der Innenfläche **27** der Kupola **7** anwählbar.

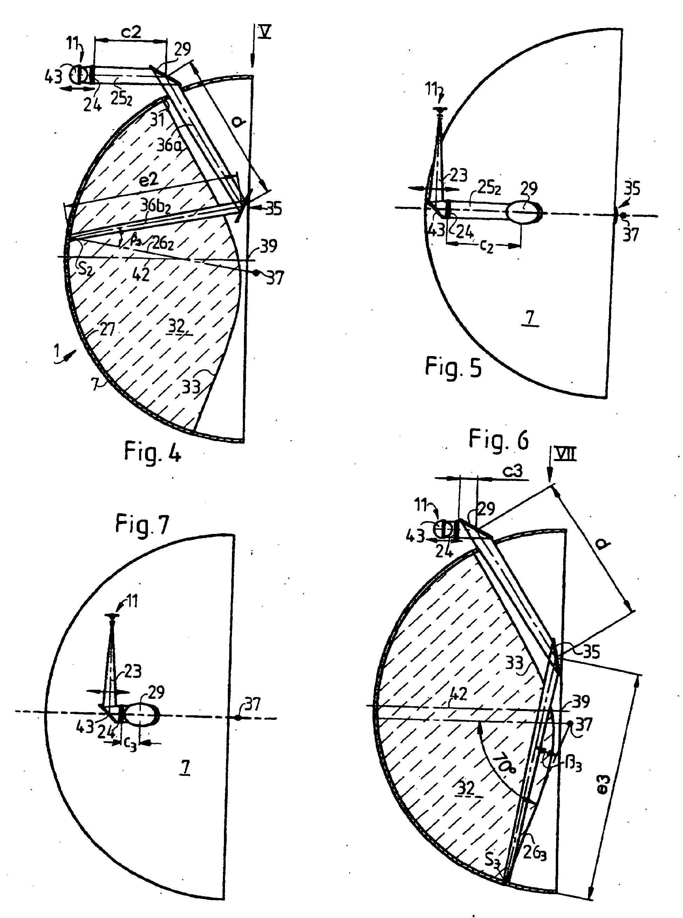

Ein Patientenauge **37**, dessen Gesichtsfeld ausgemessen werden soll, ist um einen Abstand **b** aussermittig zum Kupolamittelpunkt **39** nach unten und um einen Abstand **a** nach aussen versetzt angeordnet. Die aussermittige Lage des Patientenauges **37** nach unten liegt zwischen 5% und 10% bezüglich des Kupolaradius; und ist hier mit 7,5% vorgenommen worden. Die aussermittige Lage des Patientenauges **37** nach aussen sollte zwischen 3% und 8% betragen und liegt in der in den **Figuren 1, 4, 6, 10** und **11** gezeigten Augenlage bei 4,5%. Aufgrund der aussermittigen Anordnung des Patientenauges zum Kupolamittelpunkt **39** und der oben beschriebenen, in unmittelbarer Nachbarschaft zur Patientenstirn **21** angeordneten Schwenkspiegelanordnung **35** kann jeweils ein Winkel $\beta_1$, $\beta_2$, $\beta_3$, ... zwischen dem auf der Innenseite **27** im Ort **S** der Kupola **7** auftreffenden Lichtstrahl und einer Beobachtungsachse $26_1$ zum Patientenauge **37** klein gehalten werden, um hierdurch eine lageabhängige Verzeichnung des Stimulusquerschnitts ebenfalls klein zu halten. Einen Winkel $\beta_1$ in unmittelbarer Nähe der Aussengrenze des Gesichtsfeldes zeigt **Figur 1** und einen grösseren Winkel $\beta_2$ annähernd in der Kupolaachse **42** zeigt **Figur 4.**

Wird der Ort des Stimulus durch Verstellen der Schwenkspiegelanordnung **35** an einen anderen Ort $S_2$, wie in den **Figuren 4** und **5** gezeigt, verlegt, so ergibt sich aufgrund der aussermittigen Anordnung der Schwenkspiegelanordnung **35** eine gegenüber der Darstellung in den **Figuren 1** und **2** vergrösserte optische Weglänge als Summe der beiden Teilstrahlen **36a** und $36b_2$, welche durch die Verlängerung des Teilstrahls $36b_2$ hervorgerufen wird; die optische Weglänge d des Teilstrahls **36a** verändert sich nicht. Die Änderung der optischen Weglänge e (auch hier ist eine Durchnummerierung für die unterschiedlichen Stimuluslagen $S_n$ vorgenommen worden) des Teilstrahls **36b** wird durch eine Verschiebung der optischen Anordnung bestehend aus Lichtquelle **11,** Kondensor **13,** Blendenanordnung **15** und einem vor dem Objektiv 24 angeordneten Umlenkspiegel **43** sowie dem Objektiv **24** selbst gegenüber dem Umlenkspiegel **29** ausgeglichen. Der Abstand dieser optischen Anordnung vom Umlenkspiegel **29** wird mit $c_n$ bezeichnet und in Zuordnung zu den durchnummerierten Stimuli $S_n$ ebenfalls durchnummeriert. Mit Blick auf die beiden unterschiedlichen Stimuli $S_1$ (**Figuren 1** und **2**) und $S_2$ (**Figuren 4** und **5**) gilt nun

$$c_1 + d + e_1 = c_2 + d + e_2$$

In den **Figuren 6** und **7** ist die Projektion des Stimulus $S_3$ auf den unteren Extrempunkt dargestellt.

Als Lichtquelle **11** können diverse Quellen verwendet werden. Die Erfindung löst jedoch u.a. die Aufgabe, ein Projektionsperimeter in einer einfachen Bauart zu schaffen. Die Fachwelt hätte gemäss DIN EN ISO 12866 bis jetzt LEDs in Perimetern (und nicht in Projektionsperimetern) mit festem Raster verwendet; die Fachwelt war jedoch der Meinung, dass die ausgesandte Strahlung von Licht-Emittierenden-Dioden (LED) bei einem Projektionsperimeter nicht ausreichend sei. Es wird hier dargelegt, wie ein Einsatz von LEDs dennoch möglich ist.

Es wird davon ausgegangen, dass ein Stimulus auf der Innenfläche einer matt-weiss gestrichenen Kupola mit einer Leuchtdichte **L** von 4'000 asb = 1'274 cd/m$^2$ darzustellen sei. Da der Ort des Stimulus eine vollkommen diffuse Streuung aufweist, kann die geforderte Leuchtdichte **L** in eine zu realisierende Beleuchtungsstärke **E** bei der Projektion des Stimulus nach der Formel

$$E = L \cdot \pi / \rho$$

umgerechnet werden, wobei $\rho$ der Reflexionsgrad am Ort des Stimulus ist und mit 0,98 (matt-weiss) angenommen wird. Es wird somit eine Beleuchtungsstärke E von

$$E = 1274 \, [cd/m2] \cdot \pi / 0{,}98 \, [\Omega_0] = 4084 \, lx$$

gefordert. Mit einer 25% Sicherheit [4084 · 1,25] ergibt sich dann eine geforderte Beleuchtungsstärke von 5105 lx.

Verwendet man eine LED, wie sie beispielsweise von der Firma Lumiled unter der Bezeichnung LED LXHL-MW1D vertrieben wird, so hat diese eine Leuchtdichte von $4 \cdot 10^6$ $cd/m^2$. In dem oben beschriebenen Beispiel eines Projektionsperimeters beträgt der Projektionsabstand zwischen Objektiv **24** und Stimulus $S_n$ 665 mm. Bei einem freien Objektivdurchmesser des Objektivs **24** von **28** mm beträgt somit der bildseitige Aperturwinkel 2 · 1,206°.

Als Raumwinkel $\Omega_{St1}$ unter dem die freie Objektivfläche **A** von $14^2 \cdot \pi = 615$ $mm^2$ vom Stimulus **S** in einem Abstand **r** von 665 mm aus gesehen wird, ergibt sich gemäss der Formel

$$\Omega_{St1} = A/r^2 = 615 / 665^2 = 1{,}392 \cdot 10^{-3} \, sr.$$

Da die Leuchtdichte in einem abbildenden System im Bildraum gleich der Leuchtdichte im Objektraum ist, kann die notwendige Beleuchtungsstärke $E_{ST1}$ mit dem Verhältnis zwischen bildseitigem Raumwinkel ($1{,}392 \cdot 10^{-3}$ sr) zu objektseitigem Raumwinkel (1 sr gemäss Leuchtdichtedefinition) aus der Leuchtdichte der LED berechnet werden:

$$L_{LED} = L_{ST1} = E_{ST1} / \Omega_{St1}$$

$$E_{ST1} = L_{LED} \cdot \Omega_{St1}$$

$$E_{ST1} = 4 \cdot 10^6 \, [cd/m^2] \cdot 1{,}392 \cdot 10^{-3} \, [sr] = 5568 \, [lx]$$

Es kann somit bei der Verwendung einer LED die geforderte, eine Reserve von 25% beinhaltende Leuchtdichte von 5'105 lx erreicht werden.

Die Verwendung einer LED ergibt gegenüber der Verwendung der bekannten thermischen Lichtquelle geringere Lichtverluste, da die ausgesandte Lichtwellenlänge derart ausgewählt werden kann, dass sie möglichst der zu verwendenden Farbe des Stimulus entspricht. Besonders auffällig war beim Stand der Technik beispielsweise der geringe Wirkungsgrad, wenn vom Spektrum einer Wolframwendellampe ein blauer Stimulus erzeugt werden sollte, da Wolframwendellampen mit hoher, jedoch gegenüber LEDs geringerer Lebensdauer einen geringen Blaulichtanteil aufwiesen. Weitere Nachteile dieser Lampen waren die hohen Betriebskosten und Unannehmlichkeiten durch den relativ häufigen Lampenwechsel und die hohe Betriebstemperatur; auch wird in der Regel ein grosses Bauvolumen für die Lampenhalterung benötigt.

Der von der LED als Lichtquelle **11** ausgehende Lichtstrahl $25_n$ wird nach dem Objektiv **24** auf den betreffenden Stimulus $S_n$ abgebildet und hat im Bereich des Durchbruches **31** durch die Kupola **7** etwa einen Durchmesser von 27 mm (auf dem Objektiv **24** war ein Durchmesser von 28 mm angenommen worden). Ein Durchbruch **31** mit einem Durchmesser von 30 mm wäre somit vollständig ausreichend für einen ungehinderten Durchtritt des Strahles $25_n$. Eine derartig grosse Öffnung im Gesichtsfeld **32** wäre für dessen einwandfreie Untersuchung unzulässig. Eine derartige grosse Öffnung kann nur erreicht werden, wenn der Durchbruch **31** ausserhalb des auf die Innenseite **27** der Kupola **7** zu projizierenden Gesichtsfeldes **32** so dicht wie möglich an dessen Aussengrenze **33** angeordnet ist.

Würde man nämlich den Stimulusstrahlengang durch ein relativ kleines, nach der Messmethode geradezu noch mögliches Loch mit einem Durchmesser von maximal 18 mm im Gesichtsfeld leiten, was eine freie Öffnung am Objektiv von 17 mm nach sich zieht, so ergäbe sich mit den oben genannten Abmessungen und Daten eine Beleuchtungsstärke EST2 im Stimulus von lediglich

$$E_{ST2} = E_{ST1} \cdot (17^2/28^2) = 2'053 \, [lx],$$

was den oben genannten Anforderungen nicht entspricht.

In den **Figuren 1, 2** und **4** bis **6** ist zur Platzersparnis der von der Lichtquelle **11** ausgehende Strahl durch einen Umlenkspiegel **43** abgeknickt geführt. Durch diese Abknikkung ergibt sich eine konstruktive Platzersparnis, da die Lichtquelle **11**, der Kondensor **13** und die Blendenanordnung **15** parallel zur Vorderseite der Kupola verlaufen und das Projektionsperimeter durch diese optischen Elemente nicht nach hinten vergrössert wird. Eine Konstruktionsvariante mit eliminierten Umlenkspiegel **43** d.h. ein nicht abgeknickter Lichtstrahl, ist analog zu dem oben beschriebenen Projektionsperimeter verwendbar.

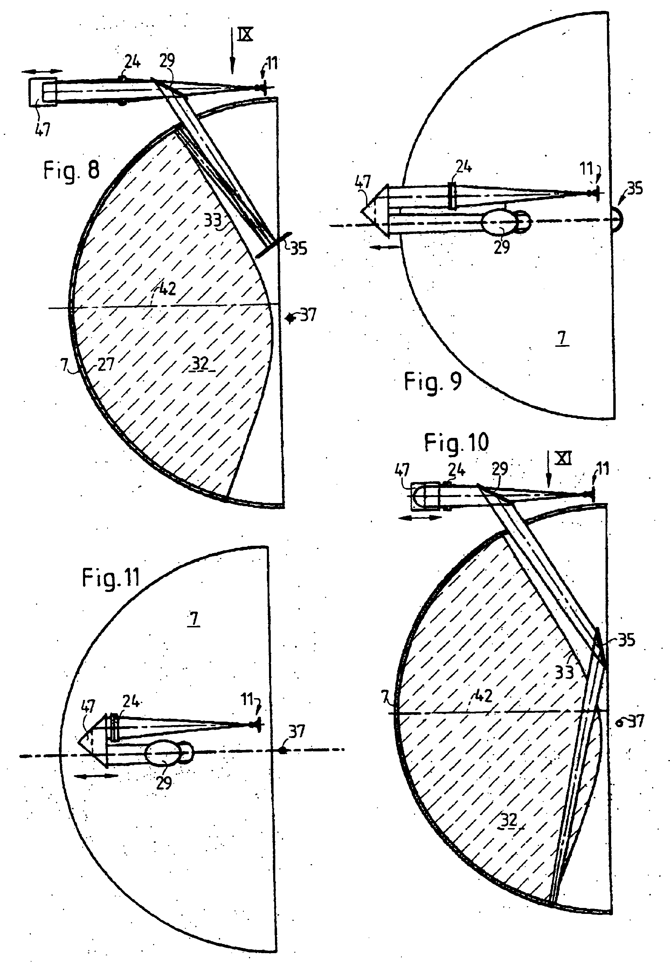

Eine weitere Platzersparnis kann durch die in den **Figuren 8** bis **11** gezeigte Ausführungsvariante erreicht werden. Entgegen der in den **Figuren 1, 2** und **4** bis **7** gezeigten Ausführungsvariante ist nun der Umlenkspiegel **43** eliminiert worden. Ein 45°-Prisma **47** wird zwischen dem Objektiv **24** und dem Umlenkspiegel **29** verwendet, wobei jetzt nur das Prisma **47** in der Richtung des aus ihn treffenden und verlassenden Strahls bewegt wird. Die lineare Bewegung entspricht dem halben Weg des zu korrigierenden Abstands zum jeweiligen Ort **S** des Stimulus.

Anstelle nur einer LED für nur eine Farbe können selbstverständlich auch mehrere auswechselbare LEDs verwendet werden, die man dann vorzugsweise auf einem Revolver einschwenkbar anordnen wird. Auch kann bei LEDs gegenüber den bekannten thermischen Lichtquellen eine Dimmung über eine Pulsweitenmodulation des Stromes vorgenommen werden. Hierdurch entfällt eine aufwändige Shutterkonstruktion. Die Ansprechzeiten von LEDs liegen < 100 ns. Auch eine Lebensdauer von 100'000 Stunden für den Einsatz der LEDs kann als Vorteil angeführt werden.

Anstelle die oben beschriebene Weglängenkorrektur unter Verwendung des Umlenkspiegels **29** oder des Umlenkprismas **47** vorzunehmen, kann insbesondere bei Verwendung von Leuchtdioden auch eine Verschiebung senkrecht zur Achse des Durchbruchs **31** vorgenommen werden, wobei man hier sogar soweit gehen kann, dass die Leuchtdiode innerhalb der Kupola **7** zu liegen kommt.

**Patentansprüche**

1. Projektionsperimeter (1) mit einem Kopfhalter (3) für einen Patientenkopf (5), mit einer hemisphärischen Kupola (7), mit einer Lichtquelle (11), von der ein Lichtstrahl (23, 25n, 36a, 36bn) aussendbar ist, und mit einer ausserhalb des Kupolamittelpunkts (39), aber innerhalb der Kupola *(7)* angeordneten Schwenkspiegelanordnung (35), mit der der Lichtstrahl (23, 25n, 36a, 36bn) auf vorgegebene Orte (Sn) auf einer Innenfläche (27) der Kupola (7) als Stimulus (Sn) zum Ausmessen eines Gesichtsfeldes (32) eines Auges (37) des Patienten führbar ist, wobei die Lichtquelle (11) ausserhalb der Kupola (7) und zwischen der Lichtquelle (11) und der Schwenkspiegelanordnung (35) ein Objektiv (24) angeordnet ist, wobei die Kupola (7) ausserhalb des Gesichtsfeldes des Patienten einen Durchbruch (31) aufweist, durch welchen der Lichtstrahl in die Kupola (7) zur Schwenkspiegelanordnung (35) führbar ist, **dadurch gekennzeichnet, dass** die Lichtquelle (11) wenigstens eine Licht-Emittierende-Diode (LED) aufweist, welche den Lichtstrahl emittiert, ein genereller Schwenkort der Schwenkspiegelanordnung (35) in einem Abstand vom Kupolamittelpunkt (39) von 15% bis 50% des Kupolaradius angeordnet ist und eine Grösse des Durchbruchs (31) zumindest so gewählt ist, dass sich eine freie Objektivfläche A ergibt, welche der Formel EST1 = LLED · A/r2 genügt, wobei LLED eine Leuchtdichte der LED, EST1 eine auf der Innenfläche der Kupola zu erreichende Beleuchtungsstärke und r einen Abstand des Stimulus von der freien Objektivfläche A bezeichnen.

2. Projektionsperimeter (1) nach Anspruch 1, **dadurch gekennzeichnet, dass** die Lichtquelle (11) mehrere Licht-Emittierende-Dioden aufweist, welche Lichtstrahlen mit zueinander unterschiedlichen Farben aussenden können, wobei die Dioden auf einer Wechseleinheit angeordnet sind, damit jeweils ein Lichtstrahl vorgegebener Farbe auf die Schwenkspiegelanordnung (35) führbar ist.

3. Projektionsperimeter (1) nach einem der Ansprüche 1 oder 2, **dadurch gekennzeichnet, dass** die Lichtquelle (11) mehrere Licht-Emittierende-Dioden ausweist, welche Lichtstrahlen mit zueinander unterschiedlichen Farben aussenden können, wobei die unterschiedliche Farben aufweisenden Lichtstrahlen mehrerer Dioden zu einem einzigen, auf die Schwenkspiegelanordnung (35) zu führenden Lichtstrahl zur Erzeugung von Mischfarben mit einer optischen Vereinigungseinheit überlagerbar sind.

4. Projektionsperimeter (1) nach einem der Ansprüche 1 oder 2, **dadurch gekennzeichnet, dass** die optische Achse (36a) des auf die Schwenkspiegelanordnung (35) treffenden Lichtstrahls gegenüber der Kupolaachse (34) einen Winkel grösser als 40°, bevorzugt grösser als 50°, hat.

5. Projektionsperimeter (1) nach einem der Ansprüche 1 bis 4, **dadurch gekennzeichnet, dass** der Durchbruch (31) in einem oberen Bereich der Kupola (7), dicht an einer Grenze (33) des Gesichtsfeldes (32) des Patienten angeordnet ist.

6. Projektionsperimeter (1) nach einem der Ansprüche 1 bis 5, **dadurch gekennzeichnet, dass** der generelle Schwenkort der Schwenkspiegelanordnung (35) oberhalb des Kupolamittelpunkts (39) in einem Abstand vom Kupolamittelpunkt (39) von 20% bis 35% des Kupolaradius, d.h. in unmittelbarer Nähe zum Ort, der mit dem Kopfhalter (3) fixierbaren Patientenstirn (21) angeordnet ist.

7. Projektionsperimeter (1) nach einem der Ansprüche 1 bis 6, **dadurch gekennzeichnet, dass** der Kopfhalter (3) derart ausgebildet ist, dass der Patientenkopf (5) derart positionierbar ist, dass das zu untersuchende Patientenauge (37) eine aussermittig, nach unten, vorzugsweise um 5% bis 10%, versetzte Lage zum Kupolamittelpunkt (39) hat, damit in Zusammenwirkung mit einer aussermittigen Schwenkspiegelanordnung (35) jeweils ein Winkel zwischen der auf der Innenseite (27) der Kupola (7) auftreffenden Achse (36bn) des Lichtstrahls und einer Beobachtungsachse (26n) zum Patientenauge (37) klein ist, wodurch eine lageabhängige Verzeichnung des Stimulusquerschnitts so klein gehalten werden kann, dass sich der ergebende Raumwinkel, unter dem der Patient den Stimulus beobachtet, innerhalb einer Toleranz von +/- 14% bleibt.

8. Projektionsperimeter (1) nach einem der Ansprüche 1 bis 7, **dadurch gekennzeichnet, dass** der Kopfhalter (3) derart ausgebildet ist, dass der Patientenkopf (5) derart positionierbar ist, dass das zu untersuchende Patientenauge (37) eine aussermittig, nach aussen, vorzugsweise um 3% bis 8%, versetzte Lage zum Kupolamittelpunkt (39) hat, damit in Zusammenwirkung mit der aussermittigen Schwenkspiegelanordnung (35) jeweils ein Winkel zwischen dem auf der Innenseite (27) der Kupola (7) auftreffenden Lichtstrahl (36bn) und einer Beobachtungsachse zum Patientenauge klein, d.h. eine lageabhängige Verzeichnung des Stimulusquerschnitts so klein gehalten werden kann, dass sich der ergebende Raumwinkel, unter dem der Patient den Stimulus beobachtet, innerhalb einer Toleranz von +/- 14% bleibt.

9. Projektionsperimeter (1) nach einem der Ansprüche 1 bis 8, **dadurch gekennzeichnet, dass** die Lichtquelle (11) ein zu ihr starr angeordnetes Strahlformungssystem (9) hat und die Lichtquelle mit dem Strahlformungssystem (9) in Richtung der optischen Achse des auf den Schwenkspiegelanordnung treffenden Lichtstrahls in Abhängigkeit des Orts des Stimulus (Sn) auf der Innenseite (27) der Kupola (7) zur Korrektur unterschiedlicher Abstände der Schwenkspiegelanordnung (35) vom jeweiligen Ort (Sn) des Stimulus verschiebbar ist.

10. Projektionsperimeter (1) nach einem der Ansprüche 1 bis 8, **dadurch gekennzeichnet, dass** die Lichtquelle (11) ein zu ihr starr angeordnetes Strahlformungssystem (9) hat und eine erste optische Weglänge (cn + d) eines ersten Lichtstrahlteilstück zwischen dem Strahlformungssystem (9) und der Schwenkspiegelanordnung (35) gemäss einer optischen Weglängenveränderung (en) eines zweiten Lichtstrahlteilstücks zwischen Schwenkspiegelanordnung (35) und Stimulus (Sn) unter Verwendung einer Strahlablenk- (43) bzw. -umkehreinheit (47) korrigierbar ist.

**Claims**

1. Projection perimeter (1) having a head holder (3) for a patient's head (5), having a hemispherical cupola (7), having a light source (11) from which a light beam (23, 25n, 36a, 36bn) is emissable, and having a pivoting mirror arrangement (35) arranged beyond the cupola midpoint (39) but within the cupola (7), with which mirror arrangement the light beam (23, 25n, 36a, 36bn) is guidable onto predetermined locations (Sn) on an inner surface (27) of the cupola (7) as stimulus (Sn) for measuring the field of vision (32) of an eye (37) of the patient, wherein the light source (11) is arranged outside the cupola (7) and a lens (24) is arranged between the light source (11) and the pivoting mirror arrangement (35), wherein outside the field of vision of the patient the cupola (7) has an opening (31) through which the light beam is guidable into the cupola (7) to the pivoting mirror arrangement (35), **characterised in that** the light source (11) comprises at least one light-emitting diode (LED) that emits the light beam, a general pivoting location of the pivoting mirror arrangement (35) is arranged at a distance from the cupola midpoint (39) of from 15% to 50% of the cupola radius and a size of the opening (31) is selected to be at least such that a free lens area A that satisfies the formula $EST1 = LLED \cdot A/r2$ is achieved, wherein LLED is a luminance of the LED, EST1 denotes an illumination level to be reached on the inner surface of the cupola and r denotes a distance of the stimulus from the free lens area A.

2. Projection perimeter (1) according to claim 1, **characterised in that** the light source (11) comprises a plurality of light-emitting diodes, which are able to emit light beams of different colours, wherein the diodes are arranged on a change unit so that each time a light beam of predetermined colour is directable onto the pivoting mirror arrangement (35).

3. Projection perimeter (1) according to either claim 1 or claim 2, **characterised in that** the light source (11) comprises a plurality of light-emitting diodes, which are capable of emitting light beams with different colours, wherein to produce mixed colours the light beams having different colours of a plurality of diodes are superimposable by means of an optical combining unit to form a single light beam leading onto the pivoting mirror arrangement (35).

4. Projection perimeter (1) according to either claim 1 or claim 2, **characterised in that** the optical axis (36a) of the light beam striking the pivoting mirror arrangement (35) has an angle greater than 40°, preferably greater than 50°, with respect to the cupola axis (34).

5. Projection perimeter (1) according to any one of claims 1 to 4, **characterised in that** the opening (31) is arranged in an upper region of the cupola (7), close to a boundary (33) of the field of vision (32) of the patient.

6. Projection perimeter (1) according to any one of claims 1 to 5, **characterised in that** the general pivoting location of the pivoting mirror arrangement (35) is arranged above the cupola midpoint (39) at a distance from the cupola midpoint (39) of from 20% to 35% of the cupola radius, i.e. in the immediate vicinity of the location of the patient's forehead (21) fixable in position with the head holder (3).

7. Projection perimeter (1) according to any one of claims 1 to 6, **characterised in that** the head holder (3) is designed in such a way that the patient's head (5) is positionable in such a manner that the patient's eye (37) to be examined has a position offset eccentrically and downwardly, preferably by 5% to 10%, with respect to the cupola midpoint (39), so that in co-operation with an eccentric pivoting mirror arrangement (35), in each case an angle between the axis (36bn) of the light beam striking the inside (27) of the cupola (7) and an observation axis (26n) to the patient's eye (37) is small, whereby a position-dependent distortion of the stimulus cross-section can be kept so small that the resulting solid angle at which the patient observes the stimulus remains within a tolerance of $\pm$ 14%.

8. Projection perimeter (1) according to any one of claims 1 to 7, **characterised in that** the head holder (3) is designed in such a way that the patient's head (5) is positionable in such a manner that the patient's eye (37) to be examined has a position offset eccentrically and outwardly, preferably by 3% to 8%, with respect to the cupola midpoint (39), so that in co-operation with the eccentric pivoting mirror arrangement (35), in each case an angle between the light beam (36bn) striking the inside (27) of the cupola (7) and an observation axis to the patient's eye is small, i.e. a position-dependent distortion of the stimulus cross-section can be kept so small that the resulting solid angle at which the patient observes the stimulus remains within a tolerance of $\pm$ 14%.

9. Projection perimeter (1) according to any one of claims 1 to 8, **characterised in that** the light source (11) has a beam-formation system (9) arranged rigidly with respect thereto and the light source with the beam formation system (9) is displaceable in the direction of the optical axis of the light beam striking the pivoting mirror arrangement as a function of the location of the stimulus (Sn) on the inside (27) of the cupola (7) for correction of different distances of the pivoting mirror arrangement (35) from the respective location (Sn) of the stimulus.

10. Projection perimeter (1) according to any one of claims 1 to 8, **characterised in that** the light source (11) has a beam-formation system (9) arranged rigidly with respect thereto and a first optical path length (cn + d) of a first light beam subportion between the beam-formation system (9) and the pivoting mirror arrangement (35) is correctable in accordance with an optical path length change (en) of a second light beam subportion between the pivoting mirror arrangement (35) and stimulus (Sn) using a beam-deflection unit (43) and beam-reversing unit (47).

**Revendications**

1. Périmètre à projection (1) comprenant un appuie-tête (3) pour la tête d'un patient (5), une coupole hémisphérique (7), une source lumineuse (11) pouvant émettre un rayon lumineux (23, 25n, 36a, 36bn), et un dispositif à miroir pivotant (35) disposé en dehors du centre de la coupole (39) mais à l'intérieur de la coupole (7), avec lequel le rayon lumineux (23, 25n, 36a, 36bn) peut être guidé sur des endroits prédéterminés (Sn) sur une surface intérieure (27) de la coupole (7) comme stimulus (Sn) en vue de mesurer un champ de vision (32) d'un oeil (37) du patient, la source lumineuse (11) étant disposée à l'extérieur de la coupole (7) et un objectif (24) étant agencé entre la source lumineuse (11) et le dispositif à miroir pivotant (35), la coupole (7) présentant en dehors du champ de vision du patient une percée (31) par laquelle le rayon lumineux peut être guidé dans la coupole (7) en direction du dispositif à miroir pivotant (35), **caractérisé en ce que** la source lumineuse (11) présente au moins une diode électroluminescente (DEL), laquelle émet le rayon lumineux, un point général de pivotement du dispositif à miroir pivotant (35) étant disposé à une distance du centre de la coupole (39), de 15 % à 50 % du rayon de la coupole, et une grandeur de la percée (31) étant au moins choisie de manière à donner une surface libre d'objectif A, laquelle répond à la formule EST1 = LDEL A/r2, LDEL désignant une luminance de la DEL, EST1 désignant une intensité lumineuse à atteindre sur la surface intérieure de la coupole et r désignant une distance du stimulus par rapport à la surface libre d'objectif A.

2. Périmètre à projection (1) selon la revendication 1, **caractérisé en ce que** la source lumineuse (11) présente plusieurs diodes électroluminescentes, lesquelles peuvent émettre des rayons lumineux de couleurs différentes les unes par rapport aux autres, les diodes étant agencées sur une unité interchangeable pour que respectivement un rayon lumineux d'une couleur prédéterminée puisse être guidé sur le dispositif à miroir pivotant (35).

3. Périmètre à projection (1) selon la revendication 1 ou 2, **caractérisé en ce que** la source lumineuse (11) présente plusieurs diodes électroluminescentes, lesquelles peuvent émettre des rayons lumineux de couleurs différentes les unes par rapport aux autres, les rayons lumineux de plusieurs diodes, lesquels présentent différentes couleurs, pouvant être superposés avec une unité optique de réunion en un seul rayon lumineux à guider sur le dispositif à miroir pivotant (35) en vue de générer des couleurs mélangées.

4. Périmètre à projection (1) selon l'une des revendications 1 ou 2, **caractérisé en ce que** l'axe optique (36a) du rayon lumineux rencontrant le dispositif à miroir pivotant (35) possède par rapport à l'axe (34) de la coupole un angle supérieur à 40°, de préférence supérieur à 50°.

5. Périmètre à projection (1) selon l'une quelconque des revendications 1 à 4, **caractérisé en ce que** la percée (31) est disposée dans une zone supérieure de la coupole (7), près d'une limite (33) du champ de vision (32) du patient.

6. Périmètre à projection (1) selon l'une quelconque des revendications 1 à 5, **caractérisé en ce que** le point général de pivotement du dispositif à miroir pivotant (35) est disposé au-dessus du centre de la coupole (39) à une distance du centre de la coupole (39), de 20 % à 35 % du rayon de la coupole, c'est-à-dire à proximité immédiate de l'endroit où le front du patient (21) peut être fixé à l'aide de l'appuie-tête (3).

7. Périmètre à projection (1) selon l'une quelconque des revendications 1 à 6, **caractérisé en ce que** l'appuie-tête (3) est conçu de manière à positionner la tête (5) du patient de telle façon que l'oeil (37) du patient à examiner possède une position décalée de manière excentrée, vers le bas, de préférence de 5 % à 10 %, par rapport au centre de la coupole (39), pour qu'en coopération avec un dispositif excentré à miroir pivotant (35) respectivement un angle faible soit ménagé entre l'axe (36bn) du rayon lumineux rencontrant la face intérieure (27) de la coupole (7) et un axe d'observation (26n) vers l'oeil du patient (37), ce qui permet de réduire une distorsion, dépendante de la position, de la section du stimulus pour que l'angle solide qui en résulte, sous lequel le patient observe le stimulus, reste à l'intérieur d'une tolérance de +/- 14%.

8. Périmètre à projection (1) selon l'une quelconque des revendications 1 à 7, **caractérisé en ce que** l'appuie-tête (3) est conçu de manière à positionner la tête (5) du patient de telle façon que l'oeil (37) du patient à examiner possède une position décalée de manière excentrée, vers l'extérieur, de préférence de 3 % à 8 %, par rapport au centre de la coupole (39), pour qu'en coopération avec un dispositif excentré à miroir pivotant (35) respectivement un angle faible soit ménagé entre le rayon lumineux (36bn) rencontrant la face intérieure (27) de la coupole (7) et un axe d'observation vers l'oeil du patient (37), c'est-à-dire qu'il est possible de réduire une distorsion, dépendante de la position, de la section du stimulus de telle manière que l'angle solide qui en résulte, sous lequel le patient observe le stimulus, reste à l'intérieur d'une tolérance de +/- 14 %.

9. Périmètre à projection (1) selon l'une quelconque des revendications 1 à 8, **caractérisé en ce que** la source lumineuse (11) possède un système de mise en forme de faisceau (9) disposé de manière rigide par rapport à celle-ci et la source lumineuse peut être déplacée avec le système de mise en forme de faisceau (9) dans le sens de l'axe optique du rayon lumineux rencontrant le dispositif à miroir pivotant en fonction de l'endroit du stimulus (Sn) sur la face intérieure (27) de la coupole (7) en vue de corriger différentes distances du dispositif à miroir pivotant (35) par rapport à l'endroit respectif (Sn) du stimulus.

10. Périmètre à projection (1) selon l'une quelconque des revendications 1 à 8, **caractérisé en ce que** la source lumineuse (11) possède un système de mise en forme de faisceau (9) disposé de manière rigide par rapport à celle-ci, et une première longueur de parcours optique (cn + d) d'un premier tronçon de rayon lumineux entre le système de mise en forme de faisceau (9) et le dispositif à miroir pivotant (35) pouvant être corrigée selon une modification de la longueur de parcours optique (en) d'un second tronçon de rayon lumineux entre le dispositif à miroir pivotant (35) et le stimulus (Sn) par utilisation d'une unité d'inversion de faisceau (47) et/ou de déviation de faisceau (43).

Fig. 1

Fig. 2

Fig. 3

EP 1 380 251 B1

Fig. 4

Fig. 5

Fig. 7

Fig. 6

14

EP 1 380 251 B1

Fig. 8

Fig. 9

Fig.10

Fig.11

15

EP 1 380 251 B1

Fig. 12

16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5235360 A
- EP 242723 A

- EP 0242723 A